# EUROPÄISCHE PATENTANMELDUNG

(11) **EP 3 785 619 A1**
(43) Veröffentlichungstag der Anmeldung: **03.03.2021**
(21) Anmeldenummer: 19194188.9
(22) Anmeldetag: 28.08.2019
(51) Int. Cl.: A61B 5/024, A61B 5/00, A61B 5/0456

(54) **VERFAHREN ZUR DETEKTION EPILEPTISCHER UND PSYCHOGENER ANFÄLLE**

(71) Anmelder: Life Science Inkubator Betriebs GmbH & Co. KG, 53175 Bonn (DE)
(72) Erfinder: Klett, Kevin, 72076 Metzingen (DE); Lutz, Florian, 70193 Stuttgart (DE); Hofmeister, Julian, 72072 Tübingen (DE)
(74) Vertreter: von Renesse, Dorothea

(57) **Zusammenfassung**

Die Erfindung betrifft ein Verfahren zur Detektion epileptischer oder psychogener Anfälle umfassend die Schritte:
a. Erfassen einer Vielzahl von zeitlich aufeinanderfolgenden Kennwerten eines Kennwerttyps, wobei ein Kennwert auf Grundlage einer Reihe zeitlich aufeinanderfolgender RR-Intervalle ermittelt wird, wobei sich die Reihen zeitlich aufeinanderfolgender Kennwerte vorzugsweise dadurch unterscheiden, dass die Reihe, auf deren Grundlage der nachfolgende Kennwert ermittelt wird, anstatt des ältesten RR-Intervalls der Reihe, auf deren Grundlage der vorhergehende Kennwert ermittelt wird (Vorgängerreihe), das zeitlich auf das jüngste RR-Intervall der Vorgängerreihe folgende RR-Intervall umfasst,
b. Vergleichen des zeitlichen Verlaufs der Kennwerte mit dem zeitlichen Verlauf von Kennwerten desselben Kennwerttyps, die gemäß Verfahrensschritt a ermittelt wurden und deren Ermittlung auf RR-Intervallen beruht, die einen Anfall indizieren (Vergleichskennwerte),
c. Identifizieren eines Anfalls, wenn der zeitliche Verlauf der Kennwerte ein einen Anfall indizierendes Charakteristikum des zeitlichen Verlaufs der Vergleichskennwerte (Verlaufscharakteristikum) aufweist.

## Beschreibung

Die Erfindung betrifft ein Verfahren zur Detektion epileptischer oder psychogener Anfälle, bei dem eine Vielzahl von zeitlich aufeinanderfolgenden Kennwerten erfasst werden und die Kennwerte auf Grundlage einer Reihe zeitlich aufeinanderfolgender RR-Intervalle ermittelt werden. Ferner betrifft die Erfindung eine Vorrichtung zur Datenverarbeitung umfassend Mittel zur Ausführung des Verfahrens. Des Weiteren betrifft die Erfindung ein System zur Detektion epileptischer oder psychogener Anfälle, das insbesondere als tragbares und mobiles Gerät ausgestaltet ist und die Vorrichtung zur Datenverarbeitung aufweist.

Es sind Verfahren bekannt, bei denen als Kennwert beispielsweise die Herzfrequenzvariabilität oder der modifizierte Cardio-Sympathische-Index verwendet und ein zeitlicher Verlauf der Kennwerte erfasst wird. Ferner wird ein Schwellenwert festgelegt, bei dessen Überschreiten die Identifikation eines Anfalls angenommen wird. Problematisch ist, dass dieser sogenannte Schwellenwertansatz patientenabhängig ist, sodass ein allgemeingültiger Schwellenwert nicht existiert. Das führt häufig dazu, dass Anfälle vermeintlich identifiziert werden, obwohl sich ein Anfall tatsächlich nicht ereignet hat. Umgekehrt kommt es auch vor, dass sich ein Anfall ereignet, die Kennwerte aber unter dem Schwellenwert liegen, sodass eine Identifikation nicht erfolgen kann. Insbesondere werden fokale epileptische Anfälle derzeit nur unzureichend detektiert, obwohl diese Anfallsart einen Großteil der epileptischen Anfälle ausmacht. Um einen Anfall zuverlässig identifizieren und wichtige Informationen über den Anfall wie die Anfallsart dokumentieren zu können, sind Patienten bisher größtenteils auf einer ärztlichen Überwachung in einem Krankenhaus angewiesen.

Aufgabe der Erfindung ist, ein Verfahren zur Detektion epileptischer und psychogener Anfälle vorzuschlagen, welches eine hohe Zuverlässigkeit hat, ohne dass sich der Patient einer ärztlichen Überwachung unterziehen muss.

Die Aufgabe wird mit dem Verfahren nach Anspruch 1 gelöst. Ferner wird die Aufgabe mit der Vorrichtung nach Anspruch 11 und dem System nach Anspruch 12 gelöst. Vorteilhafte Ausführungsformen sind in den jeweiligen Unteransprüchen und der Beschreibung sowie in den Figuren wiedergegeben.

Erfindungsgemäß umfasst das Verfahren folgende Schritte
a. Erfassen einer Vielzahl von zeitlich aufeinanderfolgenden Kennwerten eines Kennwerttyps, wobei ein Kennwert auf Grundlage einer Reihe zeitlich aufeinanderfolgender RR-Intervalle ermittelt wird, wobei sich die Reihen zeitlich aufeinanderfolgender Kennwerte vorzugsweise dadurch unterscheiden, dass die Reihe, auf deren Grundlage der nachfolgende Kennwert ermittelt wird, anstatt des ältesten RR-Intervalls der Reihe, auf deren Grundlage der vorhergehende Kennwert ermittelt wird (Vorgängerreihe), das zeitlich auf das jüngste RR-Intervall der Vorgängerreihe folgende RR-Intervall umfasst,
b. Vergleichen des zeitlichen Verlaufs der Kennwerte mit dem zeitlichen Verlauf von Kennwerten desselben Kennwerttyps, die gemäß Verfahrensschritt a ermittelt wurden und deren Ermittlung auf RR-Intervallen beruht, die einen Anfall indizieren (Vergleichskennwerte),
c. Identifizieren eines Anfalls, wenn der zeitliche Verlauf der Kennwerte ein einen Anfall indizierendes Charakteristikum des zeitlichen Verlaufs der Vergleichskennwerte (Verlaufscharakteristikum) aufweist.

Ein RR-Intervall wird vorzugsweise ermittelt, indem zwei aufeinanderfolgende R-Zacken eines Elektrokardiogramms mittels des Algorithmus von Pan und Tompkins erfasst werden und danach der Abstand zwischen den R-Zacken berechnet wird. Grundsätzlich erfolgt die Ermittlung des RR-Intervalls unmittelbar nach dem Erfassen der R-Zacken.

RR-Intervalle, die einen Anfall indizieren, sind RR-Intervalle, die zur Zeit eines Anfalls an einem Patienten, der den Anfall erlitten hat, ermittelt wurden (anfallsindizierend RR-Intervalle). In diesem Sinne bedeutet "zur Zeit eines Anfalls" der Zeitabschnitt vom Beginn des Anfalls bis zum Ende des Anfalls oder ein Teilabschnitt dieses Zeitabschnitts. Vorteilhafterweise sind auch Zeitabschnitte vor und nach dem Anfall umfasst, wenn diese eine Relevanz für weitere Informationen über einen Anfall haben können, wie zum Beispiel Schlussfolgerungen über einen herannahenden Anfall liefern können. Anfallsindizierende RR-Intervalle können beispielsweise während einer ärztlichen Überwachung des Patienten gemessen worden sein. Vorteilhafterweise beruht die Ermittlung der Vergleichskennwerte auf typischen anfallsindizierenden RR-Intervallen, die auf Grundlage einer Vielzahl anfallsindizierender RR-Intervalle ermittelt werden können. Durch eine Auswertung der Vielzahl anfallsindizierender RR-Intervalle können auch anfallsindizierende RR-Intervalle identifiziert werden, die nicht nur allgemein typisch, sondern auch spezifisch typisch für einen Anfall sind. In diesem Kontext bedeutet "spezifisch typisch", dass die betreffenden RR-Intervalle nicht nur allgemein einem Anfall zugeordnet werden können, sondern darüber hinaus auch eine spezifische Zuordnung zu weiteren Informationen ermöglichen. Solche Informationen können beispielsweise die Art eines Anfalls sein. Somit ist es nicht nur möglich, einen Anfall zu identifizieren, sondern auch die Anfallsart.

Im engeren Sinne sind unter anfallsindizierende RR-Intervallen RR-Intervalle zu verstehen, die zur Zeit eines Anfalls an einem Patienten, der den Anfall erlitten hat, ermittelt wurden (originäre anfallsindizierende RR-Intervalle). In einem weiteren Sinne umfasst der Begriff "anfallsindizierende RR-Intervalle" auch RR-Intervalle, die aus originären anfallsindizierenden RR-Intervallen abgeleitet sind (derivative anfallsindizierende RR-Intervalle). Dies kann beispielsweise durch maschinelles Lernen geschehen. So könnte beispielsweise ein Computer basierend auf einen stetig wachsenden Datensatz von anfallsindizierenden RR-Intervallen präzisere bzw. zuverlässigere anfallsindizierende RR-Intervalle generieren.

Ein Kennwert wird auf Grundlage einer Reihe zeitlich aufeinanderfolgender RR-Intervalle ermittelt. Auf welche Weise die RR-Intervalle die Ermittlung des Kennwerts bestimmen, hängt von dem Typen der Operation ab, dessen Operanden die RR-Intervalle sind. Der Operationstyp kann beispielsweise die Mittelwertberechnung sein. In diesem Fall wird die Summe der RR-Intervalle gebildet und durch die Anzahl der RR-Intervalle dividiert. Der Kennwert ist dann der Mittelwert der RR-Intervalle.

Grundsätzlich ist im Sinne dieser Erfindung "zeitlich aufeinanderfolgend" als zeitlich unmittelbar aufeinanderfolgend zu verstehen.

Die Reihen zeitlich aufeinanderfolgender Kennwerte unterscheiden sich dadurch, dass die Reihe, auf deren Grundlage der nachfolgende Kennwert ermittelt wird (Folgereihe), anstatt des ältesten RR-Intervalls der Reihe (Altintervall), auf deren Grundlage der vorhergehende Kennwert ermittelt wird (Vorgängerreihe), das zeitlich auf das jüngste RR-Intervall der Vorgängerreihe folgende RR-Intervall (Neuintervall) umfasst. Es sind auch Ausführungsformen denkbar, bei denen sich die Folgereihe von der Vorgängerreihe dadurch unterscheidet, dass die Folgereihe anstatt mehrerer Altintervalle, mehrere Neuintervalle umfasst, wobei vorzugsweise die Anzahl der Altintervalle und die Anzahl der Neuintervalle identisch sind. Es ist bevorzugt, dass jede Reihe die gleiche Anzahl von RR-Intervallen umfasst, z. B. 100 RR-Intervalle. Es ist auch denkbar, dass jede Reihe eine Anzahl von RR-Intervallen umfasst, die während einer bestimmten Zeitspanne gemessen werden. Bei der Zeitspanne kann es sich beispielsweise um 30 Sekunden handeln.

In einer alternativen Ausführungsform umfasst die Folgereihe kein RR-Intervall der Vorgängerreihe. Denkbar ist, dass die Folgereihe mit dem RR-Intervall beginnt, dass zeitlich auf das jüngste RR-Intervall der Vorgängerreihe folgt.

Im Sinne der Erfindung umfasst "Identifizieren eines Anfalls" den Fall, dass ein Anfall identifiziert wird, wenn dieser bereits begonnen hat, aber auch den Fall, dass ein Anfall zwar noch nicht begonnen hat, dessen Eintritt aber bevorsteht. Daher eignet sich das erfindungsgemäße Verfahren auch zum Warnen vor einem drohenden Anfall.

Im Sinne der Erfindung bedeutet "Vielzahl" mindestens eine Anzahl von zwei. Vorzugsweise steht "Vielzahl" für eine Anzahl, die viel größer ist als zwei, zum Beispiel mindestens 1000.

Der zeitliche Verlauf der Vergleichskennwerte umfasst das Verlaufscharakteristikum. Es ist aber auch denkbar, dass der zeitliche Verlauf der Vergleichskennwerte ausschließlich aus dem Verlaufscharakteristikum oder eines Teils dessen besteht.

In einer bevorzugten Ausführungsform umfasst das erfindungsgemäße Verfahren ferner die folgenden Schritte
d. Erfassen einer Vielzahl von zeitlich aufeinanderfolgenden Kennwerten eines zweiten Kennwerttyps (Zweitkennwerte) entsprechend Verfahrensschritt a,
e. Vergleichen des zeitlichen Verlaufs der Zweitkennwerte mit dem zeitlichen Verlauf von Kennwerten des zweiten Kennwerttyps, die entsprechend Verfahrensschritt a ermittelt wurden und deren Ermittlung auf RR-Intervallen beruht, die einen Anfall indizieren (Vergleichszweitkennwerte),
wobei ein Anfall nach Verfahrensschritt c. nur identifiziert wird, wenn zusätzlich der zeitliche Verlauf der Zweitkennwerte ein einen Anfall indizierendes Charakteristikum des zeitlichen Verlaufs der Vergleichszweitkennwerte (Verlaufscharakteristikum für Zweitkennwerte) aufweist.

Vorzugsweise sind die Reihen, auf deren Grundlage die Kennwerte ermittelt werden, und die Reihen, auf deren Grundlage die Zweitkennwerte ermittelt werden, identisch. In einer alternativen bevorzugten Ausführungsform sind die Reihen, auf deren Grundlage die Kennwerte ermittelt werden, und die Reihen, auf deren Grundlage die Zweitkennwerte ermittelt werden, nicht identisch oder nur teilidentisch. Besonders bevorzugt, werden die Kennwerte und die Zweitkennwerte zeitgleich ermittelt. An dieser Stelle sei darauf hingewiesen, dass die Buchstaben, welche die Verfahrensschritte in den Ansprüchen bezeichnen, die Verfahrensschritte nicht chronologisch ordnen, sondern lediglich einer vereinfachten Bezugnahme auf einzelne Verfahrensschritte dienen, ohne dass der Wortlaut des in Bezug genommenen Verfahrensschritts wiederholt zu werden braucht. In einer denkbaren Ausführungsform jedoch ordnen die Buchstaben gemäß ihrer alphabetischen Reihenfolge die Verfahrensschritte in den Ansprüchen chronologisch.

Die Ausführungsform gemäß Anspruch 2 hat insbesondere den Vorteil, dass die Wahrscheinlichkeit, dass ein Anfall irrtümlicherweise detektiert wird, verringert wird und ein Anfall erst detektiert wird, wenn zwei Bedingungen kumulativ erfüllt sind: Der zeitliche Verlauf der Kennwerte weist ein Verlaufscharakteristikum auf und der zeitliche Verlauf der Zweitkennwerte weist ein Verlaufscharakteristikum für Zweitkennwerte auf.

In einer alternativen Ausführungsform der Erfindung weist das Verfahren zur Detektion eines epileptischen oder psychogenen Anfalls folgende Schritte auf
- Erfassen einer Vielzahl von zeitlich aufeinanderfolgenden Kennwerten eines Kennwerttyps, wobei ein Kennwert auf Grundlage einer Reihe zeitlich aufeinanderfolgender RR-Intervalle ermittelt wird, wobei sich die Reihen zeitlich aufeinanderfolgender Kennwerte vorzugsweise dadurch unterscheiden, dass die Reihe, auf deren Grundlage der nachfolgende Kennwert ermittelt wird, anstatt des ältesten RR-Intervalls der Reihe, auf deren Grundlage der vorhergehende Kennwert ermittelt wird (Vorgängerreihe), das zeitlich auf das jüngste RR-Intervall der Vorgängerreihe folgende RR-Intervall umfasst,
- Erfassen einer Vielzahl von zeitlich aufeinanderfolgenden Kennwerten eines mindestens zweiten Kennwerttyps (Zweitkennwerte) entsprechend dem vorhergehenden Verfahrensschritt dieser alternativen Ausführungsform,
- Vergleichen des zeitlichen Verlaufs der Kennwerte mit dem zeitlichen Verlauf von Zweitkennwerten,
- Auf Grundlage des Vergleichs ermitteln, ob ein Anfall vorliegt oder nicht.

Diese alternative Ausführungsform unterscheidet sich von den übrigen Ausführungsformen dadurch, dass zum Identifizieren eines Anfalls keine Vergleichskennwerte und Vergleichscharakteristika benötigt werden.

In einer bevorzugten Ausführungsform umfasst das erfindungsgemäße Verfahren ferner die folgenden Schritte
f. Entsprechende Durchführung der Schritte d. und e. für mindestens einen dritten Kennwerttypen,
wobei ein Anfall nach Verfahrensschritt c. nur identifiziert wird, wenn zusätzlich der zeitliche Verlauf der Drittkennwerte ein einen Anfall indizierendes Charakteristikum des zeitlichen Verlaufs der Vergleichsdrittkennwerte (Verlaufscharakteristikum für Drittkennwerte) aufweist.

Der Begriff "mindestens" weist darauf hin, dass der Verfahrensschritt f. für eine beliebige Anzahl von Kennwerttypen entsprechend durchgeführt werden kann. Somit kann es zum Beispiel neben einem Drittkennwert auch einen Viertkennwert - also ein Kennwert eines vierten Kennwerttyps - geben. Folgerichtig ist auch die Bedingung dieser Ausführungsform auf einen Viertkennwert entsprechend anzuwenden. In diesem Fall würde die Bedingung lauten: "[...] wenn zusätzlich der zeitliche Verlauf der Viertkennwerte ein einen Anfall indizierendes Charakteristikum des zeitlichen Verlaufs der Vergleichsviertkennwerte (Verlaufscharakteristikum für Drittkennwerte) aufweist."

Der zweite Kennwerttyp ist ein anderer Kennwerttyp als der (erste) Kennwerttyp. Der dritte Kennwerttyp ist ein anderer als der zweite Kennwerttyp und ein anderer als der (erste) Kennwerttyp. Gleiches gilt für jeden weiteren Kennwerttypen. So ist zum Beispiel ein vierter Kennwerttyp ein anderer als der erste, zweite und dritte Kennwerttyp.

Vorzugsweise sind die Reihen, auf deren Grundlage die Kennwerte ermittelt werden, die Reihen, auf deren Grundlage die Zweitkennwerte ermittelt werden, und die Reihen, auf deren Grundlage die Drittkennwerte ermittelt werden, identisch. Es ist aber auch durchaus denkbar, dass die Reihen, auf deren Grundlage die Kennwerte ermittelt werden, und die Reihen, auf deren Grundlage die Zweitkennwerte ermittelt werden, und die Reihen auf deren Grundlage die Drittkennwerte ermittelt werden, etc. nicht identisch oder nur teilidentisch sind. Besonders bevorzugt, werden die Kennwerte, die Zweitkennwerte und die Drittkennwerte zeitgleich ermittelt.

In einer alternativen Ausführungsform dieser Ausführungsform umfasst das erfindungsgemäße Verfahren folgende Verfahrensschritte
- Entsprechende Durchführung der Schritte d. und e. für mindestens einen dritten Kennwerttypen,
wobei ein Anfall nach Verfahrensschritt c. identifiziert wird, wenn der zeitliche Verlauf der Drittkennwerte kein Verlaufscharakteristikum für Drittkennwerte aufweist.

In einer bevorzugten Ausführungsform ist der Kennwerttyp der Mittelwert, die Standardabweichung, der Cardio-Sympathische-Index (CSI), der funktional modifizierte Cardio-Sympathische-Index (CSImod), der Cardio-Vascular-Index, der Stressindex nach Baevsky, der Adäquanzindex der Regulationsprozesse, der Index des vegetativen Gleichgewichts, der vegetative Rhytmusindex, die Quadratwurzel des Mittelwerts der Summe aller quadrierten Differenzen zwischen benachbarten RR-Intervallen (RMSSD), die Schiefe der Verteilung der RR-Intervalle (RRSkew), die Wölbung der Verteilung der RR-Intervalle (RRKurt), der Prozentsatz der Intervalle mit mindestens 50 Millisekunden Abweichung vom vorausgehenden Intervall (pNN50), die Anzahl der Paare benachbarter RR-Intervalle, die mehr als 50 Millisekunden voneinander in der gesamten Aufzeichnung abweichen (NN50), der Frequenzbereiche unter 0,003 Herz (ULF), der Frequenzbereiche 0,003 - 0,04 Herz (VLF), der Frequenzbereiche 0,04 -0,15 Herz (LF), der Frequenzbereiche 0,14-0,4 Herz (HF), das Verhältnis LF zu HF, die absolute spektrale Leistungsdichte (ttlpwr), die am stärksten vertretene spektrale Leistungsdichte aus dem Low-Frequenzy Band und aus dem High-Frequenzy Band, die am stärksten vorkommende Frequenz im Low- bzw. High-Frequenzy Band, die Beschleunigungs- und Verlangsamungskapazität des Herzrhythmus basierend auf RR-Intervallen (PRSA-AC bzw. PRSA-DC), die Probenentropie (SampEn), die approximierte Entropie (ApEn), der Koeffizient der Probeentropie (CoSEN), die langfristige und kurzfristige trendbereinigende Fluktuationsanalyse (DFA), das Integral der Dichteverteilung bzw. der Triangular Index (TRI), die approximierte Länge und Breite der Ellipse des Poincare Plots sowie deren Quotient, die Herzratenturbulenz (HRT) oder die Recurrence Rate (REC).

Der zeitliche Verlauf der Kennwerte kann beispielsweise visualisiert werden, wenn die Kennwerte in einem Koordinatensystem gegen die Zeit aufgetragen werden. Dadurch kann das Verlaufscharakteristikum auch visualisiert werden. Es kann eine bestimmte Form aufweisen. In einer bevorzugten Ausführungsform ist das Verlaufscharakteristikum eine im Wesentlichen linear fallende Kurve (Linearfallen), wenn der Kennwerttyp der Mittelwert ist und eine im Wesentlichen hügelförmige Kurve (Hügelform), wenn der Kennwerttyp die Standardabweichung oder der CSI ist.

In einer bevorzugten Ausführungsform ist der Kennwerttyp der (Erst-)Kennwerte der Mittelwert, der Kennwerttyp der Zweitkennwerte die Standardabweichung und der Kennwerttyp der Drittkennwerte der CSI.

In einer bevorzugten Ausführungsform des erfindungsgemäßen Verfahrens umfasst das Verfahren ferner folgende Schritte
- Bilden zeitlich aufeinanderfolgender Intervalle, die zeitlich aufeinanderfolgende Kennwerte umfassen (Kennwertintervalle), wobei Kennwertintervalle derart zeitlich aufeinanderfolgen, dass ein nachfolgendes Kennwertintervall sich von dem vorhergehenden Kennwertintervall dadurch unterscheidet, dass es anstatt des ältesten Kennwerts des vorhergehenden Kennwertintervalls den Kennwert, der zeitlich auf den jüngsten Kennwert des vorhergehenden Kennwertintervalls folgt, umfasst,
- Kennwertintervallweise prüfen, ob das Verlaufscharakteristikum in dem jeweiligen Kennwertintervall auftritt.

Kennwertintervalle folgen derart zeitlich aufeinander, dass ein nachfolgendes Kennwertintervall sich von dem vorhergehenden Kennwertintervall dadurch unterscheidet, dass es anstatt des ältesten Kennwerts des vorhergehenden Kennwertintervalls (Altkennwert) den Kennwert, der zeitlich auf den jüngsten Kennwert des vorhergehenden Kennwertintervalls folgt (Neukennwert), umfasst. Es sind auch Ausführungsformen denkbar, bei denen sich das nachfolgende Kennwertintervall von dem vorhergehenden Kennwertintervall dadurch unterscheidet, dass das nachfolgende Kennwertintervall anstatt mehrerer Altkennwerte, mehrere Neukennwerte umfasst, wobei vorzugsweise die Anzahl der Altkennwerte und die Anzahl der Neukennwerte identisch sind. Es ist bevorzugt, dass jedes Kennwertintervall die gleiche Anzahl von Kennwerten umfasst.

In einer bevorzugten Ausführungsform des erfindungsgemäßen Verfahrens umfasst das Verfahren folgende Verfahrensschritte:
- Unterteilen der Kennwertintervalle in mindestens zwei vorzugsweise im Wesentlichen gleich große Unterintervalle
- Ermitteln eines Überkennwerts eines jeden Unterintervalls
- Auf Grundlage der Überkennwerte identifizieren, ob ein Verlaufscharakteristikum vorliegt oder nicht.

Ein Überkennwert wird auf Grundlage einer Reihe zeitlich aufeinanderfolgender Kennwerte ermittelt. Auf welche Weise die Kennwerte die Ermittlung des Überkennwerts bestimmen, hängt von dem Typen der Operation ab, dessen Operanden die Kennwerte sind. Der Operationstyp kann beispielsweise die Mittelwertberechnung sein. In diesem Fall wird die Summe der Kennwerte gebildet und durch die Anzahl der Kennwerte dividiert. Der Überkennwert ist dann der Mittelwert der Kennwerte. Der Überkennwert entspricht daher dem Kennwert. Vorzugsweise ist der Überkennwert einer der in der Anmeldung genannten Beispiele für Kennwerte (z. B. Mittelwert, CSImod, REC, etc).

In einer bevorzugten Ausführungsform des erfindungsgemäßen Verfahrens umfasst das Verfahren ferner folgende Schritte
- Unterteilen der Kennwertintervalle in vorzugsweise vier im Wesentlichen gleich große Unterintervalle
- Ermittlung des Mittelwertes aller Kennwerte eines jeden Unterintervalls
- Identifizieren des Verlaufscharakteristikum Linearfallen, wenn der Mittelwert jedes älteren Unterintervalls größer ist als der Mittelwert jedes jüngeren Unterintervalls

Die Größe eines Unterintervalls wird durch die Anzahl der Kennwerte, welche das Unterintervall umfasst, bestimmt. Im Sinne der Erfindung bedeutet "im Wesentlichen gleich große Unterintervalle", dass die Unterintervalle gleich groß sind oder dass sich ihre Größen derart unterscheiden, dass die Identifizierung des Verlaufscharakteristikums nicht beeinträchtigt wird. Im Fall der letzten Alternative kann das Verhältnis zwischen Unterintervallgröße und Durchschnittsintervallgröße als Beurteilungskriterium herangezogen werden. Ein Beispiel soll dies erläutern: Die Unterintervallgrößen betragen 101, 99. 102 und 98 Kennwerte. Die Durchschnittsintervallgröße beträgt 100 Kennwerte. Darauf bezogen weichen die Unterintervalle lediglich um 1 bis 2 % von der Durchschnittsintervallgröße ab. Dies ist ein Indiz dafür, dass die Unterintervalle gemäß der Erfindung im Wesentlichen gleich groß sind.

Das Verfahren dieser Ausführungsform kann mehrmals durchgeführt werden, wobei die Anzahl der RR-Intervalle, die von einer Reihe (Reihenlänge) umfasst sind, sich bei jeder Durchführung ändert. Diese mehrmaligen Durchführungen können gleichzeitig oder nacheinander erfolgen. Vorzugsweise beträgt die Anzahl der Durchführungen drei, wobei die Reihenlänge 50, 75, 100 RR-Intervalle beträgt. Das Ergebnis jeder Durchführung ist das Identifizieren oder Nichtidentifizieren eines Verlaufscharakteristikums. Die Ergebnisse der Durchführungen können mit Hilfe der booleschen Algebra zu logischen Aussagen verknüpft werden. So kann zum Beispiel definiert werden, dass als Gesamtergebnis ein Verlaufscharakteristikum vorliegt, wenn eines der Durchführungen das Ergebnisse liefert, dass ein Verlaufscharakteristikum identifiziert wurde.

Alternativ ist auch eine andere Anzahl von Unterintervallen denkbar.

In einer weiteren bevorzugten Ausführungsform dieser Ausführungsform des erfindungsgemäßen Verfahrens umfasst das Verfahren ferner folgende Schritte
- Unterteilen der Kennwertintervalle in vier im Wesentlichen gleich große Unterintervalle,
- Ermittlung des Mittelwertes aller Kennwerte eines jeden Unterintervalls
- Unterteilen des ältesten Unterintervalls in vier gleich große Unterintervalle (Unterunterintervalle) und ermitteln des Mittelwerts aller Kennwerte eines jeden Unterunterintervalls,
- Für das älteste Unterintervall prüfen, ob der Mittelwert jedes jüngeren Unterunterintervall größer ist als der Mittelwert jedes älteren Unterunterintervalls (Linearsteigen des ältesten Unterunterintervalls),
- Unterteilen des jüngsten Unterintervalls in vier gleich große Unterintervalle (Unterunterintervalle) und ermitteln des Mittelwerts aller Kennwerte eines jeden Unterunterintervalls,
- Für das jüngste Unterintervall prüfen, ob der Mittelwert jedes älteren Unterunterintervall größer ist als der Mittelwert jedes jüngeren Unterunterintervalls (Linearfallen des jüngsten Unterunterintervalls),
- Identifizieren des Verlaufscharakteristikum Hügelform, wenn
   - ein Linearsteigen des ältesten Unterunterintervalls vorliegt,
   - ein Linearfallen des jüngsten Unterunterintervalls vorliegt,
   - der Mittelwert des ältesten Unterintervalls kleiner ist als der Mittelwert des zweitältesten Unterintervalls und
   - der Mittelwert des zweitjüngsten Unterintervalls größer ist als der Mittelwert des jüngsten Unterintervalls.

Das Verfahren dieser Ausführungsform kann mehrmals durchgeführt werden, wobei die Anzahl der RR-Intervalle, die von einer Reihe (Reihenlänge) umfasst sind, sich bei jeder Durchführung ändert. Diese mehrmaligen Durchführungen können gleichzeitig oder nacheinander erfolgen. Vorzugsweise beträgt die Anzahl der Durchführungen vier, wobei die Reihenlänge 50, 75, 100 und 150 RR-Intervalle beträgt. Das Ergebnis jeder Durchführung ist das Identifizieren oder Nichtidentifizieren eines Verlaufscharakteristikums. Die Ergebnisse der Durchführungen können mit Hilfe der booleschen Algebra zu logischen Aussagen verknüpft werden. So kann zum Beispiel definiert werden, dass als Gesamtergebnis ein Verlaufscharakteristikum vorliegt, wenn eines der Durchführungen das Ergebnisse liefert, dass ein Verlaufscharakteristikum identifiziert wurde.

Das erfindungsgemäße Verfahren eignet sich zur Überwachung eines Patienten, sodass eine zeitnahe Alarmierung erfolgen kann, sobald ein Anfall identifiziert wurde. In diesem Fall ist es angezeigt, dass nach dem Ermitteln der RR-Intervalle die Verfahrensschritte unmittelbar und zügig ausgeführt werden. Günstig für eine zeitnahe Alarmierung ist es daher, wenn die Reihen eine geringere Anzahl von RR-Intervallen umfassen. In einer vorteilhaften Ausführungsform umfasst eine Reihe 4 bis 300 RR-Intervalle.

Das erfindungsgemäße Verfahren eignet sich auch zur Analyse von Anfällen. In diesem Fall können auch RR-Intervalle verwendet werden, deren Ermittlung vor einer längeren Zeit erfolgte. Mit Hilfe des erfindungsgemäßen Verfahrens kann dann nicht nur festgestellt werden, ob und wie oft der Patient in der Vergangenheit einen Anfall erlitten hatte, sondern es kann auch die Art des Anfalls identifiziert werden. Für Analysezwecke ist es daher vorteilhaft, wenn die Reihen eine größere Anzahl von RR-Intervallen umfassen. In einer bevorzugten Ausführungsform umfasst eine Reihe 4 bis 5000 RR-Intervalle.

Die Erfindung betrifft auch eine Vorrichtung zur Datenverarbeitung, die Mittel zur Ausführung des erfindungsgemäßen Verfahrens aufweist. Bei der Vorrichtung zur Datenverarbeitung kann es sich zum Beispiel um einen Computer oder ein Smartphone handeln.

Die Erfindung betrifft ferner ein System zur Detektion epileptischer oder psychogener Anfälle, welche die erfindungsgemäße Vorrichtung zur Datenverarbeitung aufweist. Ferner umfasst dieses System einen Sensor zum Erfassen von Herzschlagdaten sowie eine Ausgabeeinheit zur Mitteilung von Informationen über die Detektion. Durch den Sensor zum Erfassen von Herzschlagdaten können RR-Intervalle durch direkte Messung am Patienten ermittelt werden. Dadurch kann das System zur Überwachung eines anfallgefährdeten Patienten benutzt werden. Die Ausgabeeinheit teilt dem Benutzer (z. B. dem Patienten oder einem Arzt) Informationen über einen detektierten Anfall, zum Beispiel das Auftreten eines Anfalls, die Anfallsart und der Zeitpunkt des Anfalls, mit. Dadurch kann zum Beispiel ein Alarm ausgelöst werden. Es können aber auch Informationen in einem Anfallskalender gespeichert werden. Der Speicherort des Anfallskalenders kann sich beispielsweise in der Vorrichtung zur Datenverarbeitung befinden.

In einer bevorzugten Ausführungsform des erfindungsgemäßen Systems weist das System ferner einen Sender zum Senden von Informationen über die Detektion auf. Der Sender dient beispielsweise dazu, einen Notruf oder Alarm abzusetzen, wenn ein Anfall detektiert wurde.

In einer bevorzugten Ausführungsform ist das System als tragbares und mobiles Gerät ausgebildet. Die tragbare und mobile Ausgestaltung des Gerätes kann beispielsweise nach dem Vorbild eines mobilen EKG-Gerätes erfolgen. Das Gerät kann beispielsweise am Handgelenk oder an der Brust des Patienten befestigt werden. Dadurch kann der Patient auch ohne Anwesenheit eines Arztes und ohne an eine Krankenhausaufnahme gebunden zu sein, zuverlässig überwacht werden.

Die Erfindung wird nachstehend an beispielhaften Ausführungsformen erläutert.

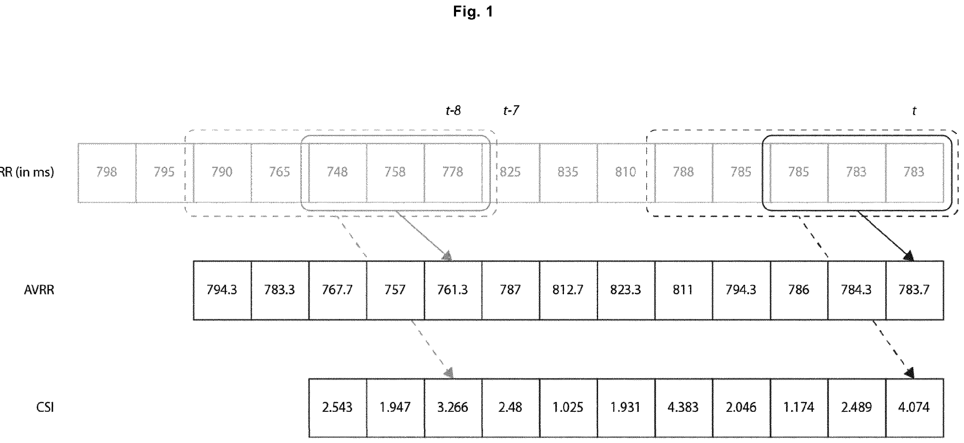

Figur 1 veranschaulicht, wie zeitlich aufeinanderfolgende Kennwerte ermittelt werden. Zu sehen sind drei Zeilen, eine obere, eine mittlere und eine untere. In der oberen Zeile sind die konkret ermittelten RR-Intervalle eingetragen. Es kann sich dabei beispielsweise um RR-Intervalle handeln, die an einem anfallgefährdeten Patienten gemessen werden, um zu überwachen, ob dieser an einem Anfall leidet oder ein Anfall bevorsteht. In dem Beispiel gemäß Figur 1 sind 15 RR-Intervalle gezeigt (15 Kästchen). In jedem Kästchen ist der jeweilige Wert des entsprechenden RR-Intervalls in der Einheit Millisekunde (ms) angegeben. Zum Beispiel hat das erste RR-Intervall den Wert 798 ms und das zweite den Werte 795 ms. Die gezeigten RR-Intervalle sind zeitlich aufeinander folgend. Dabei ist das äußerst linke RR-Intervall (798 ms) das zeitlich älteste der gezeigten RR-Intervalle (es wurde also als erstes gemessen) und das äußerst rechte RR-Intervall (783 ms) das jüngste (es wurde also als letztes gemessen). In der mittleren Zeile sind die Kennwerte des Kennwerttypen Mittelwert eingetragen. Zum Zeitpunkt *t* - *also dem Zeitpunkt, in dem das jüngste RR-Intervall gemessen wurde* - *hat* der Mittelwert auf Grundlage der letzten Reihe zeitlich aufeinanderfolgender RR-Intervalle den Wert 783,7. Die Reihe umfasst hierbei zur Veranschaulichung eine Länge von 3 RR-Intervallen. Der Mittelwert 783,7 ist also der Mittelwert der jüngsten drei RR-Intervalle 785, 783 und 783. Zur Veranschaulichung hat in der letzten Zeile der Kennwerttyp CSI zum selbigen Zeitpunkt t den Wert 4.074, der auf Grundlage der jüngsten fünf RR-Intervalle (788, 785, 785, 783 und 783) ermittelt wurde. Die Reihe, auf deren Grundlage der Zweitkennwert (hier CSI) ermittelt wird, ist also mit fünf RR-Intervallen größer als die Reihe, auf deren Grundlage der (Erst-)Kennwert (Mittelwert) ermittelt wird, die lediglich drei RR-Intervalle enthält. Die Reihenlänge der verschiedenen Kennwerte ist in diesem Beispiel verschieden.

Der Mittelwert zum Zeitpunkt t-8 (das RR-Intervall hat hier den Wert 778 ms und der Mittelwert beträgt 761,3 ms) wird ebenfalls auf Grundlage einer Reihe von drei RR-Intervallen ermittelt, nämlich 748, 758 und 778 (diese sind mittels eines Rechtecks mit durchgezogenen Linien umrandet). Die Reihen für die Ermittlung jedes Mittelwerts (mittlere Zeile) ist also in Figur 1 drei RR-Intervalle lang. Die Reihe, auf deren Grundlage der Mittelwert bei t-8 ermittelt wird, ist die Vorgängerreihe für die Reihe, auf deren Grundlage der Mittelwert bei t-7 ermittelt wird, die wiederum Folgereihe dieser Vorgängerreihe ist. Diese Folgereihe umfasst die RR-Intervalle 758, 778 und 825. Damit wandert die Reihe, die im vorliegenden Beispiel eine konstante Länge von drei RR-Intervallen hat, bildlich gesprochen kästchenweise von links nach rechts. Dies setzt sich im vorliegenden Beispiel so lange fort, bis die letzte in Figur 1 sichtbare Reihe erreicht ist (RR-Intervalle 785, 783 und 783).

Nach diesem Prinzip der wandernden Reihe werden auch die CSI-Werte ermittelt. Beispielsweise hat der CSI im Zeitpunkt t-8 den Wert 3.266, der auf Grundlage der fünf RR-Intervalle, die mittels eines Rechtecks mit gestrichelten Linien umrandet sind, ermittelt wird (RR-Intervalle 790, 765, 748, 758, 778). Auch die Reihe, die der Ermittlung des Zweitkennwerts CSI zu Grunde liegt, hat in diesem Beispiel immer eine konstante Länge (im Unterschied zum Erstkennwert aber die Länge 5). Auch diese Reihe wandert kästchenweise nach rechts bis die letzte sichtbare Reihe erreicht wird, die aus den RR-Intervallen 788, 785, 785, 783, 783 besteht. Bei jedem Schritt des Weiterwanderns wird ein CSI-Wert ermittelt (siehe unterste Zeile).

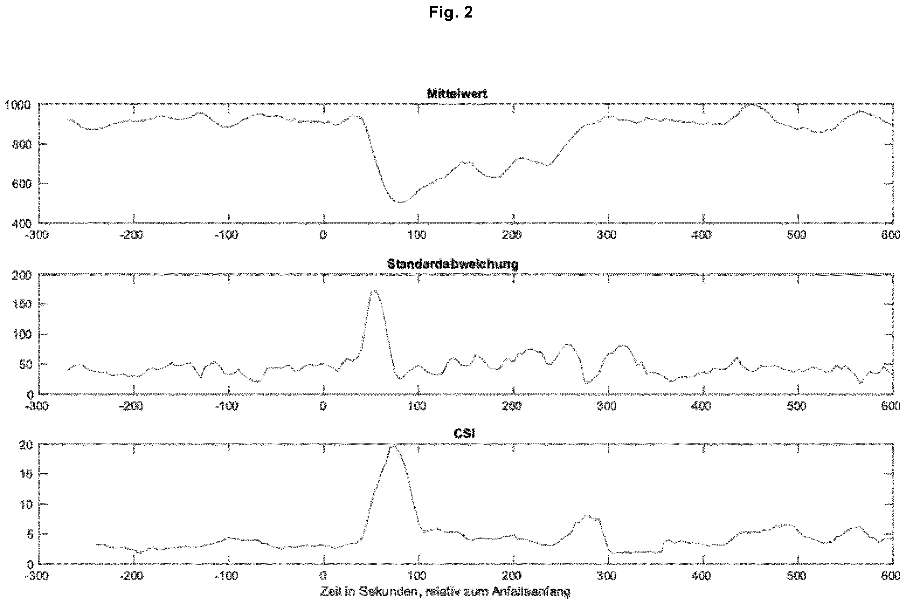

Figur 2 zeigt den zeitlichen Verlauf einer Vielzahl von zeitlich aufeinanderfolgenden Kennwerten eines ersten Kennwerttyps (oberer Graph), den zeitlichen Verlauf einer Vielzahl von zeitlich aufeinanderfolgenden Kennwerten eines zweiten Kennwerttyps (Graph in der Mitte) und den zeitlichen Verlauf einer Vielzahl von zeitlich aufeinanderfolgenden Kennwerten eines dritten Kennwerttyps (unterer Graph). Der erste Kennwerttyp ist der Mittelwert, der zweite Kennwerttyp ist die Standardabweichung und der dritte Kennwerttyp ist der CSI. Deutlich zu sehen sind die Verlaufscharakteristika, die alle samt bei einer Zeit von ungefähr 40 Sekunden verortet sind. Die RR-Intervalle, die zu dieser Zeit erfasst sind, indizieren einen Anfall.

Übereinstimmend weisen alle drei Graphen an dieser Stelle ein Verlaufscharakteristikum auf. Das Verlaufscharakteristikum der Kennwerte des Kennwerttyps Mittelwert (oben) ist ein Linearfallen, während das Verlaufscharakteristikum der Kennwerte des Kennwerttyps Standardabweichung (Mitte) und des Kennwerttyps CSI (unten) eine Hügelform ist.

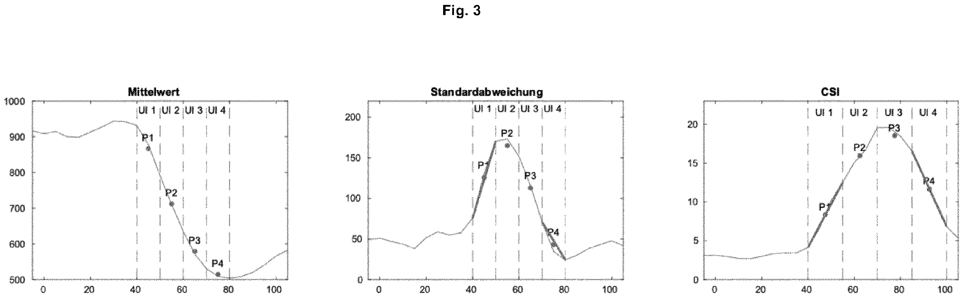

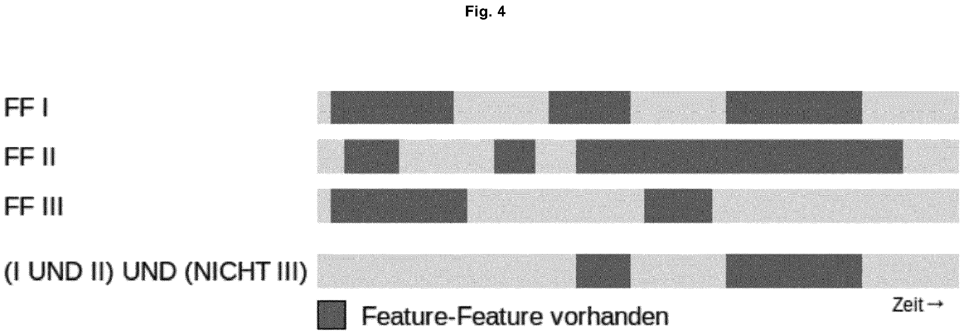

Figur 3 veranschaulicht für die Kennwerte dreier Kennwerttypen (linke Teilfigur: Mittelwert; mittlere Teilfigur: Standardabweichung; rechte Teilfigur: CSI) die Unterteilung der Kennwertintervalle in vier Unterintervalle (UI1, UI2, UI3, UI4). Auf Basis der vier Unterintervalle werden am Beispiel des Kennwerttyps Mittelwert alle Mittelwerte der Kennwerte eines jeden Unterintervalls ermittelt (P1, P2, P3, P4). (Zum besseren Verständnis sei darauf hingewiesen, dass sich das Kennwertintervall in diesem Beispiel von 40 bis 80 Sekunden erstreckt.) Auf dieser Grundlage ist das Identifizieren des Verlaufscharakteristikum Linearfallen möglich, wenn der Mittelwert jedes älteren Unterintervalls größer ist als der Mittelwert jedes jüngeren Unterintervalls, wie in der linken Teilfigur (Grafik Mittelwert) dargestellt. Für die Kennwerttypen Standardabweichung (mittlere Teilfigur) und CSI (rechte Teilfigur) wird das Verlaufscharakteristikum Hügelform wie folgt identifiziert: Unterteilen in vier im Wesentlichen gleichgroße Unterintervalle und Prüfen, ob für ältestes Unterintervall (UI1) das Verlaufscharakteristikum Linearsteigen vorliegt, ob für jüngstes Unterintervall (UI4) das Verlaufscharakteristikum Linearfallen vorliegt, ob der Mittelwert des ältesten Unterintervalls (UI1) kleiner ist als der Mittelwert des zweitältesten Unterintervalls (UI2) und der Mittelwert des zweitjüngsten Unterintervalls (UI3) größer ist als der Mittelwert des jüngsten Unterintervalls (UI4). Das Ermitteln des Verlaufscharakteristikums Linearsteigen im ältesten Unterintervall (UI1) erfolgt durch Unterteilung des Unterintervalls in vier gleich große Unterunterintervalle (nicht dargestellt); für jedes Unterunterintervall wird der Mittelwert ermittelt und das Verlaufscharakteristikum Linearsteigen identifiziert, wenn der Mittelwert jedes älteren Unterunterintervalls kleiner ist als der Mittelwert jedes jüngeren Unterunterintervalls. Entsprechendes gilt für die Ermittlung des Verlaufscharakteristikums Linearfallen für das jüngste Unterintervall (UI4). Es sei der vollständigkeitshalber darauf hingewiesen, dass sich das Kennwertintervall in der mittleren Teilfigur von 40 bis 80 Sekunden erstreckt und jenes in der rechten Teilfigur von 40 bis 100. Das Ergebnis jeder Durchführung ist das Identifizieren oder Nichtidentifizieren eines Verlaufscharakteristikums. Die Ergebnisse der Durchführungen können mit Hilfe der booleschen Algebra zu logischen Aussagen verknüpft werden (Figur 4).

Figur 4 zeigt ein Beispiel für eine logische Verknüpfung von Detektionsergebnissen verschiedener Kennwerttypen. Im Folgenden wird zunächst Bezug genommen auf die obersten drei Zeilen. Jede Zeile steht für die Identifikation eines anderen Verlaufscharakteristikums. Beispielsweise zeigt die obere Zeile (FF I), ob das Verlaufscharakteristikum Linearfallen (Kennwerttyp Mittelwert) identifiziert wurde, die zweite Zeile von oben (FF II), ob das Verlaufscharakteristikum Hügel des Kennwerttyps Standardabweichung identifiziert wurde und die dritte Zeile von oben (FF III), ob das Verlaufscharakteristikum Hügel des Kennwerttyps CSI identifiziert wurde. Ob ein Verlaufscharakteristikum identifiziert wurde und über welchen Zeitraum zeigen die dunklen Balken und deren Länge an. Die untere Zeile zeigt als Endergebnis an, ob ein Anschlag erfolgt ist und über welchen Zeitraum. Die oberen drei Zeilen sind mit der unteren Zeile über eine logische Operation verbunden, die im Beispiel der Figur 4 folgendermaßen lautet: Nur wenn ein Verlaufscharakteristikum in FF I und ein Verlaufscharakteristikum in FF II identifiziert wird und zusätzlich auch kein Verlaufscharakteristikum in FF III identifiziert wird, wird in der unteren Zeile ein Anschlag identifiziert.

## Patentansprüche

1. Verfahren zur Detektion epileptischer oder psychogener Anfälle umfassend die Schritte:
a. Erfassen einer Vielzahl von zeitlich aufeinanderfolgenden Kennwerten eines Kennwerttyps, wobei ein Kennwert auf Grundlage einer Reihe zeitlich aufeinanderfolgender RR-Intervalle ermittelt wird, wobei sich die Reihen zeitlich aufeinanderfolgender Kennwerte vorzugsweise dadurch unterscheiden, dass die Reihe, auf deren Grundlage der nachfolgende Kennwert ermittelt wird, anstatt des ältesten RR-Intervalls der Reihe, auf deren Grundlage der vorhergehende Kennwert ermittelt wird (Vorgängerreihe), das zeitlich auf das jüngste RR-Intervall der Vorgängerreihe folgende RR-Intervall umfasst,
b. Vergleichen des zeitlichen Verlaufs der Kennwerte mit dem zeitlichen Verlauf von Kennwerten desselben Kennwerttyps, die gemäß Verfahrensschritt a ermittelt wurden und deren Ermittlung auf RR-Intervallen beruht, die einen Anfall indizieren (Vergleichskennwerte),
c. Identifizieren eines Anfalls, wenn der zeitliche Verlauf der Kennwerte ein einen Anfall indizierendes Charakteristikum des zeitlichen Verlaufs der Vergleichskennwerte (Verlaufscharakteristikum) aufweist.

2. Verfahren nach Anspruch 1, umfassend die folgenden Schritte d.
Erfassen einer Vielzahl von zeitlich aufeinanderfolgenden Kennwerten eines zweiten Kennwerttyps (Zweitkennwerte) entsprechend Verfahrensschritt a,
e. Vergleichen des zeitlichen Verlaufs der Zweitkennwerte mit dem zeitlichen Verlauf von Kennwerten des zweiten Kennwerttyps, die entsprechend Verfahrensschritt a ermittelt wurden und deren Ermittlung auf RR-Intervallen beruht, die einen Anfall indizieren (Vergleichszweitkennwerte),
wobei ein Anfall nach Verfahrensschritt d. nur identifiziert wird, wenn zusätzlich der zeitliche Verlauf der Zweitkennwerte ein einen Anfall indizierendes Charakteristikum des zeitlichen Verlaufs der Vergleichszweitkennwerte (Verlaufscharakteristikum für Zweitkennwerte) aufweist.

3. Verfahren nach Anspruch 2, umfassend zusätzlich folgende Schritte
f. Entsprechende Durchführung der Schritte d. und e. für mindestens einen dritten Kennwerttypen,
wobei ein Anfall nach Verfahrensschritt c. nur identifiziert wird, wenn zusätzlich der zeitliche Verlauf der Drittkennwerte ein einen Anfall indizierendes Charakteristikum des zeitlichen Verlaufs der Vergleichsdrittkennwerte (Verlaufscharakteristikum für Drittkennwerte) aufweist oder
wobei ein Anfall nach Verfahrensschritt c. identifiziert wird, wenn der zeitliche Verlauf der Drittkennwerte kein Verlaufscharakteristikum für Drittkennwerte aufweist.

4. Verfahren nach einem der vorigen Ansprüche, wobei der Kennwerttyp der Kennwerttyp der Mittelwert, die Standardabweichung, der Cardio-Sympathische-Index (CSI), der funktional modifizierte Cardio-Sympathische-Index (CSImod), der Cardio-Vascular-Index, der Stressindex nach Baevsky, der Adäquanzindex der Regulationsprozesse, der Index des vegetativen Gleichgewichts oder der vegetative Rhytmusindex ist.

5. Verfahren nach Anspruch 4, wobei
• das Verlaufscharakteristikum eine im Wesentlichen linear fallende Kurve ist (Linearfallen), wenn der Kennwerttyp der Mittelwert ist und
• das Verlaufscharakteristikum eine im Wesentlichen hügelförmige Kurve ist (Hügelform), wenn der Kennwerttyp die Standardabweichung oder der CSI ist.

6. Verfahren nach einem der Ansprüche 4 oder 5, soweit sich auf Anspruch 3 beziehend, wobei der Kennwerttyp der (Erst-)Kennwerte der Mittelwert ist, der Kennwerttyp der Zweitkennwerte die Standardabweichung ist und der Kennwerttyp der Drittkennwerte der CSI ist.

7. Verfahren nach einem der vorherigen Ansprüche, umfassend folgende Verfahrensschritte:
• Bilden zeitlich aufeinanderfolgender Intervalle, die zeitlich aufeinanderfolgende Kennwerte umfassen (Kennwertintervalle), wobei Kennwertintervalle derart zeitlich aufeinanderfolgen, dass ein nachfolgendes Kennwertintervall sich von dem vorhergehenden Kennwertintervall dadurch unterscheidet, dass es anstatt des ältesten Kennwerts des vorhergehenden Kennwertintervalls den Kennwert, der zeitlich auf den jüngsten Kennwert des vorhergehenden Kennwertintervalls folgt, umfasst,
• Kennwertintervallweise prüfen, ob das Verlaufscharakteristikum in dem jeweiligen Kennwertintervall auftritt.

8. Verfahren nach dem vorigen Anspruch, umfassend folgende Verfahrensschritte
• Unterteilen der Kennwertintervalle in mindestens zwei vorzugsweise im Wesentlichen gleich große Unterintervalle
• Ermitteln eines Überkennwerts eines jeden Unterintervalls
• Auf Grundlage der Überkennwerte identifizieren, ob ein Verlaufscharakteristikum vorliegt oder nicht.

9. Verfahren nach Anspruch 7 oder 8, umfassend folgende Verfahrensschritte
• Unterteilen der Kennwertintervalle in vorzugsweise vier im Wesentlichen gleich große Unterintervalle
• Ermittlung des Mittelwertes aller Kennwerte eines jeden Unterintervalls
• Identifizieren des Verlaufscharakteristikum Linearfallen, wenn der Mittelwert jedes älteren Unterintervalls größer ist als der Mittelwert jedes jüngeren Unterintervalls

10. Verfahren nach Anspruch 7, 8 oder 9, umfassend folgende Verfahrensschritte:
• Unterteilen der Kennwertintervalle in vier im Wesentlichen gleich große Unterintervalle,
• Ermittlung des Mittelwertes aller Kennwerte eines jeden Unterintervalls
• Unterteilen des ältesten Unterintervalls in vier gleich große Unterintervalle (Unterunterintervalle) und ermitteln des Mittelwerts aller Kennwerte eines jeden Unterunterintervalls,
• Für das älteste Unterintervall prüfen, ob der Mittelwert jedes jüngeren Unterunterintervall größer ist als der Mittelwert jedes älteren Unterunterintervalls (Linearsteigen des ältesten Unterunterintervalls),
• Unterteilen des jüngsten Unterintervalls in vier gleich große Unterintervalle (Unterunterintervalle) und ermitteln des Mittelwerts aller Kennwerte eines jeden Unterunterintervalls,
• Für das jüngste Unterintervall prüfen, ob der Mittelwert jedes älteren Unterunterintervall größer ist als der Mittelwert jedes jüngeren Unterunterintervalls (Linearfallen des jüngsten Unterunterintervalls),
• Identifizieren des Verlaufscharakteristikum Hügelform, wenn
∘ ein Linearsteigen des ältesten Unterunterintervalls vorliegt,
∘ ein Linearfallen des jüngsten Unterunterintervalls vorliegt,
∘ der Mittelwert des ältesten Unterintervalls kleiner ist als der Mittelwert des zweitältesten Unterintervalls und
∘ der Mittelwert des zweitjüngsten Unterintervalls größer ist als der Mittelwert des jüngsten Unterintervalls.

11. Verfahren nach einem der vorherigen Ansprüche, wobei eine Reihe 4 bis 300 oder 4 bis 5000 RR-Intervalle umfasst.

12. Vorrichtung zur Datenverarbeitung umfassend Mittel zur Ausführung des Verfahrens nach einem der vorigen Ansprüche.

13. System zur Detektion epileptischer oder psychogener Anfälle, aufweisend
• eine Vorrichtung zur Datenverarbeitung nach Anspruch 12,
• einen Sensor zum Erfassen von Herzschlagdaten,
• Ausgabeeinheit zur Mitteilung von Informationen über die Detektion.

14. System nach Anspruch 13, aufweisend einen Sender zum Senden von Informationen über die Detektion.

15. System nach Anspruch 13 oder 14, ausgestaltet als tragbares und mobiles Gerät.
